(19) Europäisches Patentamt
European Patent Office
Office européen des brevets

(11) Numéro de publication : **0 003 456**
**B1**

(12) **FASCICULE DE BREVET EUROPÉEN**

(45) Date de publication du fascicule du brevet : **07.11.84**

(51) Int. Cl.³ : **C 07 J 41/00**, A 61 K 31/585

(21) Numéro de dépôt : **79400039.8**

(22) Date de dépôt : **23.01.79**

(54) **Dérivés d'amino-3-cardénolides, procédé pour leur préparation, et médicaments les contenant.**

(30) Priorité : **27.01.78 FR 7802390**

(43) Date de publication de la demande : **08.08.79 Bulletin 79/16**

(45) Mention de la délivrance du brevet : **07.11.84 Bulletin 84/45**

(84) Etats contractants désignés : **BE CH DE FR GB IT LU NL SE**

(56) Documents cités :
**FR-A- 2 085 722**
**FR-A- 2 100 951**
**FR-A- 2 310 766**

(73) Titulaire : **ETABLISSEMENTS NATIVELLE S.A.**
**27, rue de la Procession**
**F-75015 Paris (FR)**

(72) Inventeur : **Jarreau, François-Xavier**
**5 rue Louis Hervé**
**F-78000 Versailles (FR)**
Inventeur : **Koenig, Jean-Jacques**
**1, rue Rodin**
**F-91380 Chilly-Mazarin (FR)**

(74) Mandataire : **L'Helgoualch, Jean et al**
**OFFICE PICARD 134 Boulevard de Clichy**
**F-75018 Paris (FR)**

EP 0 003 456 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention, réalisée dans les laboratoires du CERES (Centre Européen de Recherches Scientifique), concerne de nouveaux dérivés de cardénolides et plus particulièrement des amino-amides d'amino-3 cardénolides ainsi que leurs sels minéraux ou organiques, leur préparation, et leurs applications en thérapeutique.

De nombreuses substances naturelles dérivées des hétérosides cardiotoniques sont utilisées en thérapeutique pour le traitement de l'insuffisance cardiaque, et par exemple, on sait que l'activité cardiotonique des cardénolides-glycosides tels que la digitoxine, dépend notamment de la structure de la partie cardénolide et de la nature de la chaîne sucrée greffée en 3 β. Cependant ces substances naturelles présentent généralement une marge thérapeutique faible qui rend leur utilisation délicate, d'où l'intérêt de préparer des composés de structure voisine possédant une forte activité cardiotonique associée à une faible toxicité.

On a ainsi proposé de préparer des dérivés de cardénolides portant un groupe amino directement rattaché au carbone en position 3 du noyau stéroïdique, à partir des oxo-3 cardénolides par réaction avec l'hydroxylamine pour former l'oxime correspondante que l'on réduit ensuite par hydrogénation catalytique. On connaît également d'autres composés obtenus en greffant un substituant approprié en 3 de la partie cardénolide, et par exemple un reste aminé. De tels composés sont décrits dans les brevets français 2.085.722, 2.181.694 et 2.100.951. Ainsi, le brevet français 2.085.722 décrit des cardénolides substitués en 3 par un groupe —$HNR_9$, où $R_9$ est un atome d'hydrogène, ou un groupe acyle ou benzoyle ; le brevet français 2.100.951 décrit des cardénolides et bufadiénolides portant en position 3 un groupe amino primaire, secondaire ou tertiaire, ou 2-oxo-3-oxazolidinyle ou uréido ; les dérivés décrits au brevet français 2.181.694 sont des aminoesters reliés au cardénolide en position 3 par une liaison ester. Ces dérivés sont présentés comme possédant des propriétés cardiotoniques.

Toutefois la présence du reste aminé confère à ces composés des propriétés basiques.

On connaît aussi des dérivés de cardénolides tels que des esters, amino-acides de cardénolides, obtenus par couplage de génines cardiotoniques et dediacides aminés, comme décrit au brevet français 2.310.766.

La présente invention a pour objet de nouveaux dérivés de cardénolides cardiotoniques possédant une activité thérapeutique modifiée et présentant des propriétés amphotères procurant des propriétés pharmacocinétiques nouvelles et originales.

Les dérivés de cardénolides selon l'invention peuvent être représentés par la formule générale (I) suivante :

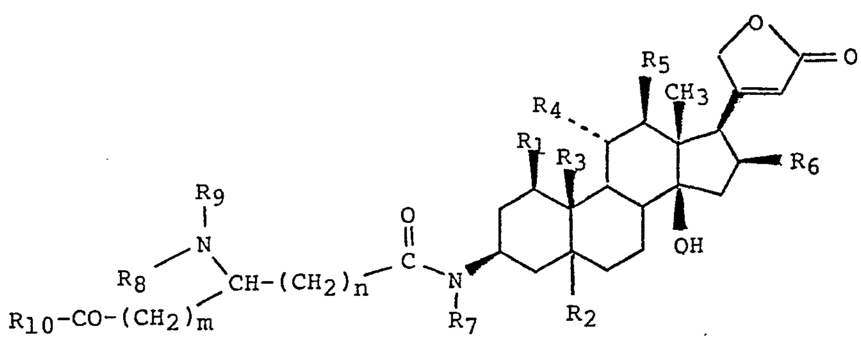

dans laquelle m et n, identiques ou différents, représentent un entier de 0 à 4, $R_1$, $R_2$, $R_4$ et $R_6$ représentent un atome d'hydrogène ou un groupe hydroxy ; $R_3$ représente un groupe méthyle, hydroxyméthyle, ou formyle ; $R_5$ représente un atome d'hydrogène, un groupe hydroxy ou un groupe acétoxy ; $R_7$ représente un atome d'hydrogène ou un groupe alkyle inférieur de 1 à 5 atomes de carbone ; $R_8$ représente un atome d'hydrogène, un groupe alkyle inférieur de 1 à 5 atomes de carbone, acyle, alkyloxycarbonyle, ou aralcoxycarbonyle ; $R_9$ représente un atome d'hydrogène ou un groupe alkyle inférieur de 1 à 5 atomes de carbone ; $R_8$ et $R_9$ peuvent former un hétérocycle conjointement avec l'atome d'azote ; $R_{10}$ représente un groupe hydroxy, alcoxy, ou aralcoxy. Dans cette formule, les substituants de la partie cardénolide ont la configuration β, à l'exception de $R_4$ qui est en α et de $R_2$ qui peut être en α ou en β.

L'invention concerne également un procédé de préparation des nouveaux dérivés de cardénolides de formule (I), consistant à faire réagir un amino-3 cardénolide par la fonction amine, avec un diacide aminé, par l'une des fonctions acide carboxylique.

Comme indiqué ci-dessus, l'activité cardiotonique des composés tels que ceux représentés par la formule (I) dépend notamment de la structure de la partie cardénolide, et plus particulièrement de la stéréochimie des substituants qui y sont greffés.

Parmi les composés représentés par la formule (I) ci-dessus, l'invention concerne de préférence les

composés pour lesquels n prend la valeur 1 ou 2, et m la valeur 0, ou inversement, $R_1$, $R_2$ et $R_6$ représentent un atome d'hydrogène ou un groupe hydroxy, $R_3$ représente un groupe méthyle, hydroxyméthyle, ou formyle, $R_4$ représente un atome d'hydrogène, un groupe hydroxy ou un groupe acétoxy.

Dans la formule (I) représentée ci-dessus, $R_7$, $R_8$ et $R_9$ représentent de préférence un atome d'hydrogène ou un groupe méthyle. Lorsque $R_8$ représente un groupe acyle, ce groupe peut être par exemple un groupe acétyle, ou un groupe acyle dérivant d'un aminoacide ou d'un oligo-peptide ; $R_8$ peut également être un groupe alkyloxy- ou aralcoxy-carbonyle, et par exemple un groupe t-butyloxycarbonyle, ou benzyloxycarbonyle diversement substitué sur le noyau aromatique ; $R_8$ et $R_9$ peuvent être conjointement deux restes acyles et former avec l'atome d'azote un groupe phtalimide ; $R_{10}$ peut représenter un groupe hydroxy, un groupe alcoxy tel que méthoxy, éthoxy ou phtalimidoéthyloxy, ou un groupe aralcoxy et notamment un groupe benzyloxy.

L'invention concerne tout particulièrement les dérivés d'amino-3 cardénolites et de l'acide aspartique ou glutamique, ainsi que leurs sels, et de préférence les composés suivants :

Composé A désoxy-3 α-L-aspartylamino-3β digitoxigénine.
Composé B désoxy-3 β-L-aspartylamino-3β digitoxigénine.
Composé C désoxy-3 α-L-glutamylamino-3β digitoxigénine.
Composé D désoxy-3 N-β-L-aspartyl N'-méthylamino-3β digitoxigénine.
Composé E désoxy-3 N-γ-L-glutamyl N'-méthylamino-3β digitoxigénine.
Composé F désoxy-3 β-L-aspartylamino-3β acétoxy-12β digoxigénine.
Composé G désoxy-3 γ-L-glutamylamino-3β acétoxy-12β digoxigénine.

Comme indiqué ci-avant, les dérivés d'amino-3 cardénolides conformes à la présente invention présentent l'avantage de posséder des propriétés amphotères résultant de la présence d'un groupe acide et d'un groupe amino basique dans la même molécule, et permettant la préparation de sels, tant par réaction avec des bases qu'avec des acides minéraux et organiques.

La présente invention concerne également les sels des amino-amides d'amino-3 cardénolides de formule I, en particulier les sels pharmaceutiquement acceptables, obtenus par réaction avec des acides usuels tels que les acides chlorhydrique, sulfurique, phosphorique, acétique, propionique, oxalique, lactique, citrique, tartrique, ascorbique, aspartique, glutamique ou malonique, ou avec des bases telles qu'un hydroxyde alcalin, et par exemple l'hydroxyde de sodium, de potassium ou de lithium, ou hydroxyde alcalino-terreux, tel que l'hydroxyde de magnésium ou de calcium. On peut également préparer des sels de métaux tels que l'aluminium, ou des sels d'ammonium.

Les sels peuvent être obtenus de façon usuelle, en faisant réagir en proportions sensiblement stoechiométriques le dérivé d'amino-3 cardénolide sous forme d'acide ou de base libre, avec un hydroxyde ou un acide approprié, dans un solvant convenablement choisi en fonction de l'acide ou de la base, par exemple l'eau ou un alcool.

Conformément au procédé de l'invention on fait réagir un amino-cardénolide, préalablement protégé le cas échéant, et un dérivé de diacide aminé convenablement substitué au niveau du groupe amino et au niveau de l'une des deux fonctions acide carboxylique, l'autre étant judicieusement activée. Comme cardénolide de départ, on peut choisir par exemple la désoxy amino-3 digitoxigénine, la désoxy amino-3 digoxigénine, la désoxy amino-3 uzarigénine, etc. Comme exemples de diacides aminés utilisables pour la préparation des composés suivant l'invention on peut citer l'acide aspartique, l'acide glutamique, l'acide amino-2 propanedioïque-1,3, l'acide amino-adipique, l'acide aminopimélique etc., sous forme lévogyre, dextrogyre, ou racémique. Ces divers acides aminés peuvent être éventuellement substitués sur l'atome d'azote.

Les groupements protecteurs du reste amino et de la fonction acide carboxylique peuvent être ceux communément employés en synthèse peptidique ; on peut citer à titre d'exemples les groupements benzyloxycarbonyle, trityle, phtalimido, etc., pour le reste amino, et les groupements ester benzylique ou méthylique, ou phtalimidométhyle, etc., pour la fonction acide carboxylique.

L'activation de la seconde fonction acide carboxylique est réalisable selon les techniques habituelles de la synthèse peptidique, en la transformant par exemple en chlorure d'acyle, ester actif, anhydride, etc., ou en l'associant à un agent de condensation tel que, par exemple, le dicyclohexyl-carbodiimide, le N,N'-carbonyl diimidazole, le diéthyl-phosphonyl cyanide, le diphénylphosphorylazide, le disulfure de triphénylphosphine ou un sel d'halogéno-2 méthylpyridinium.

De plus, les groupements protecteurs des diverses fonctions alcool, acide carboxylique, amine et/ou aldéhyde, existant dans les produits de condensation peuvent être éliminés, puis éventuellement remplacés par d'autres groupements et ce par les techniques usuelles.

Le couplage est effectué, de préférence, en ajoutant à froid l'amino-cardénolide à une solution de l'acide convenablement protégé et activé, puis en laissant la réaction évoluer à température ambiante pendant quelques heures. Il se forme avec prédominance un composé que l'on extrait par un solvant organique du milieu réactionnel préalablement dilué par de l'eau et acidifié, et que l'on purifie par cristallisation ou chromatographie.

Les expérimentations pharmacologiques et toxicologiques effectuées sur les composés de l'invention montrent qu'ils possèdent d'intéressantes propriétés permettant leur application en thérapeutique, plus particulièrement dans le domaine d'application des hétérosides cardiotoniques connus.

Plus précisément, les composés suivant l'invention possèdent une activité inhibitrice de l'ATPase $Na^+$ $K^+$ dépendante, et une activité inotrope.

L'activité inhibitrice de l'ATPase $Na^+$, $K^+$ dépendante peut être vérifiée sur le tissu de cerveau de rat. L'activité inotrope a été testée sur le cœur de cobaye isolé et perfusé (type Langendorff) et sur le cœur de chien *in situ*. Ces expérimentations montrent que les composés suivant l'invention possèdent des propriétés analogues à celles des hétérosides cartiodoniques connus bien que possédant un reste peptidique en position 3 du noyau stéroïdique, en entraînant une augmentation de la contractilité du myocarde, et en faisant apparaître en outre quelques différences au niveau des paramètres pharmacologiques ainsi qu'une intéressante marge thérapeutique.

Les doses actives, par voie intraveineuse chez le chien, sont inférieures au mg/kg de poids.

Ces propriétés pharmacologiques montrent que les composés suivant l'invention sont particulièrement indiqués pour le traitement des affections cardiaques, notamment l'insuffisance cardiaque et les troubles du rythme.

Les nouveaux composés suivant l'invention peuvent être administrés sous les formes usuelles contenant une quantité pharmacologiquement efficace du composé en tant que principe actif dilué dans les supports pharmaceutiquement utilisables, par exemple sous forme de comprimés, gélules, capsules, dragées, suppositoires, solutés injectables ou sirops.

Comme diluant solide pour la préparation des comprimés, on peut utiliser le lactose, le mannitol, le sorbitol, l'amidon, la polyvinylpyrrolidone, le stéarate de magnésium ou d'aluminium, la poudre de cellulose, la silice colloïdale, le talc, etc.

Des solutions injectables peuvent être préparées au moyen de diluants tels que l'eau bi-distillée, le propylèneglycol, une solution hydroalcoolique, ou un mélange de ces diluants, de préférence en présence d'un conservateur approprié choisi parmi ceux utilisés communément dans la technique.

Des formes buvables peuvent également être préparées, par exemple des solutés contenant le composé de l'invention dissous dans de l'eau et du glycérol en présence d'un édulcorant et d'un antioxydant, ou des suspensions du composé de l'invention dans une solution aqueuse de saccharose en présence d'un épaississant, d'un édulcorant et d'un antioxydant.

Toutes les formulations adaptées aux divers modes d'administration, c'est-à-dire par voie orale, parentérale ou rectale, peuvent être utilisées, le médicament étant associé en tant que principe actif aux excipients pharmaceutiquement acceptables convenablement choisis.

A titre d'exemple on peut citer les formulations suivantes :

| | |
|---|---|
| A — Composé A | 0,25 mg |
| Lactose | 134,75 |
| Talc | 15,0 |
| | 150,0 mg |

| | |
|---|---|
| B — Composé D | 0,25 mg |
| Amidon | 81,25 |
| Silice colloïdale | 0,50 |
| Cellulose microcristalline | 18,00 |
| | 100,0 mg |

Soluté injectable :

| | |
|---|---|
| Composé A | 0,01 mg |
| Conservateur | 0,001 |
| Eau, q.s.p. | 1,0 ml |

Soluté buvable :

| | |
|---|---|
| Composé D | 0,25 g |
| édulcorant | 2,— |
| glycérol | 30,— |
| antioxydant | 0,01 |
| eau q.s.p. | 100 ml |

La posologie peut varier selon le sujet traité et l'affection en cause, les doses administrées journellement étant généralement comprises entre 0,01 et 1 mg pour l'administration orale chez l'homme.

Les exemples suivants illustrent l'invention sans en limiter la portée.

Exemple 1

Désoxy-3 α-(N-benzyloxycarbonyl, β-benzyl)-L)-aspartyl-amino-3β digitoxigénine.

On met en solution 270 mg de désoxy-3 amino-3β digitoxigénine et 165 mg de dicyclohexylcarbodiimide dans 10 ml de chlorure de méthylène à 0°. On ajoute à cette solution, goutte à goutte en agitant à 0 °C, 297 mg d'acide N-benzyloxycarbonyl-β-benzyl-(L)-aspartique, en solution dans 10 ml de chlorure de méthylène. Après quelques minutes, un précipité blanc apparaît. Au bout d'1 heure la réaction étant terminée, on filtre, distille à sec et reprend le résidu par 20 ml de benzène. La fraction benzénique est lavée trois fois, par 10 ml d'acide chlorhydrique N, par de l'eau, par une solution de bicarbonate de sodium et enfin par de l'eau. On extrait à nouveau les fractions aqueuses par 2 fois 10 ml de benzène. On sèche et distille à sec les fractions benzéniques, on les reprend par un mélange benzène/éther (1/1), on les filtre et on distille le filtrat. Le résidu est constitué de 540 mg de désoxy-3 α-(N-benzyloxycarbonyl,β-benzyl)-(L)-aspartylamino-3β digitoxigénine, (Rdt. = 96 %).

Spectre IR (nujol) : 3 480, 3 340, 1 780, 1 755, 1 740, 1 665, 1 620 et 1 530 $cm^{-1}$

CCM : ($CH_2Cl_2$/MeOH, 95/5) : Rf = 0,47.

Exemple 2

Désoxy-3 α-L-aspartylamino-3β digitoxigénine, Composé A.

On hydrogénolyse 500 mg de désoxy-3 α(N-benzyloxycarbonyl β-benzyl)-L-aspartylamino-3β digitoxigénine, en solution dans 80 ml de méthanol, en présence de 150 mg de palladium à 5 % sur du carbonate de calcium pendant 4 h 30 (le catalyseur est renouvelé une fois). On filtre, distille à sec et on fait cristalliser le résidu (333 mg) dans l'éthanol absolu. On obtient 248 mg de cristaux de désoxy-3 α-L-aspartylamino-3β digitoxigénine, Composé A. (Rdt. = 75 %).

Point de fusion : F (Köfler) : 235-245° (dec.)

Spectre IR (Nujol) ν : 3 520, 3 275, 3 080, 1 792, 1 757, 1 726, 1 716, 1 660, 1 630, 1 620 et 1 565 $cm^{-1}$.

Spectre de RMN ($CD_3OD$) δ = 0,88 et 1,00 (2 s, $CH_3$), 2,70 (3H), 4,18 (2H), 4,99 (2H), 5,92 (1H). ppm

CCM : ($CHCl_3$/EtOH/$NH_4OH$, 60/45/15): Rf = 0,58.

Chlorhydrate du Composé A

Ce chlorhydrate est obtenu de manière usuelle par action de l'acide chlorhydrique sur le composé A, en proportions stoechiométriques.

Spectre IR (Nujol) ν : 3 370, 3 210 ; 3 050 ; 1 780 ; 1 765 ; 1 735 ; 1 678 ; 1 620 et 1 550 $cm^{-1}$.

Exemple 3

Désoxy-3 β-(N-benzyloxycarbonyl, α-benzyl)-L-aspartylamino-3β digitoxigénine.

On ajoute goutte à goutte, une solution de 430 mg d'acide (N-benzyloxycarbonyl-α-benzyl)-L-aspartique, dans 15 ml de chlorure de méthylène, à une solution, sous agitation à 0°, de 360 mg de désoxy-3 amino-3β digitoxigénine, et de 227 mg de dicyclohexylcarbodiimide dans 15 ml de chlorure de méthylène. Après une nuit de réaction à 4 °C, on filtre le précipité de dicyclohexylurée et on distille le filtrat à sec. Le résidu est repris par le mélange acétate d'éthyle/benzène (1/1), la fraction insoluble est filtrée et le filtrat est lavé par de l'acide chlorhydrique N, de l'eau, une solution de bicarbonate de sodium, enfin de l'eau. On sèche et distille à sec la phase organique. Le résidu (757 mg) est constituée de désoxy-3 β-(N-benzyloxycarbonyl-α-benzyl)-L-aspartylamino-3β digitoxigénine, contenant un peu de dicyclohexylurée (Rdt. = 100 %).

Spectre IR (Nujol) ν : 3 340, 1 780, 1 755, 1 740, 1 655, 1 535 et 1 500 $cm^{-1}$.

CCM : ($CH_2Cl_2$/MeOH, 95/5) Rf = 0,4.

Exemple 4

Désoxy-3 β-L-aspartylamino-3β-digitoxigénine, Composé B.

L'hydroxygénolyse de 600 mg de désoxy-3-β(N-benzyloxycarbonyl-α-benzyl)-L-aspartylamino-3-β digitoxigénine (c'est-à-dire le produit de l'exemple 3) en solution dans 120 ml de méthanol, en présence de 150 mg de palladium à 5 % sur carbonate de calcium pendant 90 minutes, fournit, après filtration, distillation à sec et cristallisation du résidu (413 mg) dans l'éthanol, 362 mg de désoxy-3 β-L-aspartylamino-3β digitoxigénine, Composé B, (Rdt. = 86 %).

Point de fusion F (Köfler) : 260-264 °C (dec.).

Spectre IR (Nujol) ν : 3 450, 3 340, 3 145, 3 065, 1 745, 1 650, 1 620, 1 573 et 1 510 $cm^{-1}$.

Chlorhydrate du Composé B

Spectre IR (Nujol) $\nu$ : 3 360, 3 250, 3 050, 1 780, 1 755, 1 740, 1 642, 1 627 et 1 550 $cm^{-1}$.
CCM : ($CHCl_3$/EtOH/$NH_4OH$, 50/45/15) Rf = 0,44.

Exemple 5

Désoxy-3 γ-(N benzyloxycarbonyl-α-benzyl)-L-glutamylamino-3β digitoxigénine.

On ajoute goutte à goutte une solution de 430 mg d'acide (N-benzyloxycarbonyl-α-benzyl)-L-glutamique, en solution dans 15 ml de chlorure de méthylène, à une solution de 360 mg de désoxy-3 amino-3 β digitoxigénine, et de 277 mg de dicyclohexylcarbodiimide dans 15 ml de chlorure de méthylène, sous agitation à 0°. Après 7 heures de réaction à 4°, on filtre et distille le filtrat à sec. Le résidu (860 mg) est repris par l'acétate d'éthyle, le filtrat est filtré et lavé successivement par de l'acide chlorhydrique N, de l'eau, une solution saturée de bicarbonate de sodium, enfin de l'eau. On sèche et distille à sec la phase oganique ; le résidu (732 mg) est constitué de désoxy-2 γ-(N-benzyloxycarbonyl-α-benzyl)-L-glutamylamino-3β digitoxigénine, contenant un peu de dicyclohexylurée (Rdt. = 100 %).
IR (Nujol) : 3 480, 3 340, 3 190, 1 780, 1 755, 1 740, 1 720, 1 660, 1 535 $cm^{-1}$.
CCM ($CH_2Cl_2$/MeOH-95/5) : Rf = 0,33.

Exemple 6

Désoxy-3 γ-L-glutamylamino-3β digitoxigénine, (Composé C).

L'hydrogénolyse de 548 mg de désoxy-3γ (N-benzyloxycarbonyl-α-benzyl)-L-glutamylamino-3β digitoxigénine obtenue comme indiqué dans l'exemple 5, en solution dans 100 ml de méthanol en présence de 135 mg de palladium à 5 % sur carbonate de calcium, pendant 3 heures, (le catalyseur étant renouvelé une fois) fournit, après filtration, distillation à sec et cristallisation du résidu (384 mg) dans le mélange éthanol/propanol-2 (50/50) ; 240 mg de cristaux de désoxy-3 γ-L-glutamylamino-3β digitoxigénine, Composé C. (Rdt. = 65 %).
F (Kofler) : 208-210 °C (dec.)
Spectre IR (Nujol) : 3 520, 3 340, 3 280, 3 190, 3 080, 1 790, 1 755, 1 738, 1 730, 1 635, 1 548 $cm^{-1}$.
Spectre RMN ($CD_3OD$) : δ = 0,87 et 0,97 (2 s, $CH_3$) 2,03 (2H), 2,75 (1H), 3,58 (1H), 4,05 (1H), 5,87 (S, 1H) ppm
CCM ($CHCl_3$/EtOH/$NH_4OH$, 50/45/15) : Rf = 0,47.

Chlorhydrate du Composé C

Spectre IR (Nujol) : 3 350, 1 780, 1 755, 1 740, 1 625, 1 542 $cm^{-1}$.

Exemple 7

Désoxy-3 N-β-(N-benzyloxycarbonyl-α-benzyl)-L-aspartyl N'-méthylamino-3β digitoxigénine.

On ajoute goutte à goutte une solution de 350 mg d'acide (N-benzyloxycarbonyl-α-benzyl)-L-aspartique, dans 15 ml de chlorure de méthylène, à une solution, sous agitation à 0°, de 319 mg de désoxy-3 N-méthylamino-3β digitoxigénine, et 185 mg de dicyclohexylcarbodiimide dans 15 ml de chlorure de méthylène.

Après 5 heures de réaction à 4 °C, on filtre et distille le filtrat à sec. Le résidu est repris par l'acétate d'éthyle, l'insoluble est filtré et le filtrat est lavé par de l'acide chlorhydrique N, de l'eau, une solution saturée de bicarbonate de sodium, enfin par de l'eau. On sèche et distille à sec la phase organique, et on lave plusieurs fois le résidu (605 mg) par de l'hexane. L'insoluble (572 mg) est constituée de désoxy-3 N-β-benzyloxycarbonyl-α-benzyl)-L-aspartyl N'-méthylamino-3β digitoxigénine, (Rdt. = 95 %).
Spectre IR (Nujol) : 3 430, 1 780, 1 755, 1 745, 1 630, 1 495 $cm^{-1}$.
CCM : ($CH_2Cl_2$/MeOH-97,5/2,5 atmosphère $NH_3$) Rf = 0,53.

Exemple 8

Désoxy-3 N-β-L-aspartyl N'-méthylamino-3β digitoxigénine, (Composé D).

L'hydroxygénolyse de 480 mg de désoxy-3 N-β(N-benzyl-oxycarbonyl-α-benzyl)-L-glutamylamino-3β digitoxigénine en solution dans 50 ml de méthanol, en présence de 120 mg de palladium à 5 % sur carbonate de calcium pendant 3 heures (le catalyseur étant renouvelé une fois) fournit, après filtration et distillation à sec, 60 mg de résidu cristallin blanc. On extrait plusieurs fois les filtres et l'insoluble par du

méthanol bouillant on filtre à chaud et on distille à sec. Le second résidu (240 mg), joint au premier, est lavé par de l'éthanol bouillant. Cela permet d'obtenir 161 mg de cistaux de désoxy-3 N-β-L-aspartyl N'-méthylamino-3β digitoxigénine, Composé D (Rdt. = 48 %).

F (Kofler) : 202-204 °C.

Spectre IR (Nujol) ν : 3 290, 3 175, 1 745, 1 655, 1 637, 1 618 $cm^{-1}$.

CCM ($CHCl_3$/EtOH/$NH_4OH$, 60/45/15) Rf = 0,43.

Chlorhydrate du Composé D

Spectre IR (Nujol ν : 3 400, 3 160, 1 780, 1 755, 1 625 $cm^{-1}$.

Spectre de RMN ($CD_3OD$) : δ = 0,85 et 1,07 (2 s, $CH_3$), 2,55 (1H), 3,02 ($CH_3$) 2,6 à 3,2 (3H), 4,27 (1H), 5,92 (1H) ppm.

Exemple 9

Désoxy-3 N-γ-(N-benzyloxycarbonyl-α-benzyl)-L-glutamyl N'-méthylamino-3β digitoxigénine.

On ajoute goutte à goutte une solution de 356 mg d'acide (N-benzyloxycarbonyl-α-benzyl)-L-glutamique en solution dans 15 ml de chlorure de méthylène, à une solution, sous agitation à 0 °C, de 308 mg de désoxy-3 méthylamino-3β digitoxigénine et de 182 mg de dicyclohexylcarbodiimide dans 15 $cm^3$ de chlorure de méthylène. Après 24 heures de réaction à 4 °C, on filtre et distille à sec. Le résidu est repris par de l'acétate d'éthyle, lavé par de l'acide chlorhydrique N, de l'eau, une solution saturée de bicarbonate de sodium, enfin de l'eau. Après extraction des phases aqueuses trois fois avec de l'acétate d'éthyle de la même manière, on sèche et distille à sec les phases organiques. Le résidu (693 mg) est lavé plusieurs fois avec de l'hexane. L'insoluble (516 mg) est constitué de désoxy-3 N-γ-(N-benzyloxycarbonyl, α-benzyl)-L-glutamyl, N'-méthylamino-3β digitoxigénine (Rdt. = 86 %).

Spectre IR (Nujol) ν : 3 430, 3 300, 1 780, 1 755, 1 740, 1 725, 1 620, 1 525, 1 495 $cm^{-1}$.

CCM : ($CH_2/Cl_2$/MeOH, 97,5/2,5, atmosphère $NH_3$) Rf = 0,37.

Exemple 10

Désoxy-3 N-γ-L-glutamyl, N'-méthylamino-3β digitoxigénine, (Composé E).

L'hydroxygénolyse de 560 mg de désoxy-3 N-γ-(N-benzyloxycarbonyl-α-benzyl)-L-glutamyl n'-méthylamino-3β digitoxigénine en solution dans 50 ml de méthanol en présence de 120 mg de palladium à 5 % sur carbonate de calcium pendant 4 heures (le catalyseur étant renouvelé une fois), permet d'obtenir, après filtration, distillation à sec du filtrat et cristallisation deux fois du résidu (356 mg) dans un mélange propanol-2 acétate d'éthyle, et lavage des cristaux à l'éther, 156 mg de cristaux de désoxy-3 N-γ-L-glutamyl, N'-méthylamino-3β digitoxigénine, Composé E. (Rdt. = 40 %).

F (Kofler) : 188-192 °C (dec.).

Spectre IR (Nujol) ν : 3 430, 1 780, 1 755, 1 745, 1 720, 1 620 $cm^{-1}$.

Spectre de RMN ($CD_3OD$) δ : 0,88-1,00 et 3,0 (3s, $CH_3$), 2,3 à 2,9 (3H) 3,9 (1H), 4,45 (1H) 5,87 (1H) ppm.

CCM : ($CHCl_3$/EtOH/$NH_4OH$, 60/45/15) Rf = 0,4.

Exemple 11

Désoxy-3 β-(N-benzyloxycarbonyl, α-benzyl)-L-aspartylamino-3β acétoxy-12β digoxigénine.

On ajoute goutte à goutte une solution de 430 mg d'acide (N-benzyloxycarbonyl, α-benzyl)-L-aspartique dans 15 ml de chlorure de méthylène à une solution, sous agitation à 0°, de 430 mg de désoxy-3 amino-3β acétoxy 12β digoxigénine et de 227 mg de dicyclohexylcarbodiimide dans 15 ml de chlorure de méthylène. Après 8 heures de réaction à 4 °C, le précipité est filtré et le filtrat distillé à sec. Le résidu est repris par de l'acétate d'éthyle, l'insoluble est filtré et le filtrat lavé par de l'acide chlorhydrique N, de l'eau, une solution de bicarbonate de sodium, enfin de l'eau.

Après séchage et distillation à sec de la phase organique, le résidu (812 mg) est repris plusieurs fois par le mélange acétate d'éthyle-benzène (1/1) à chaud ; on filtre et distille le filtrat. Le résidu (767 mg) est constitué de désoxy-3 β-(N-benzyloxycarbonyl, α-benzyl)-L-aspartylamino-3β acétoxy-12β digoxigénine (Rendement = 100 %).

Spectre IR (Nujol) ν : 3 460, 3 340, 1 790, 1 755, 1 740, 1 650, 1 620, 1 530 $cm^{-1}$.

CCM : ($CH_2Cl_2$/MeOH, 95/5) Rf = 0,38.

Exemple 12

Désoxy-3 β-L-aspartylamino-3β acétoxy-12β digoxigénine, (Composé F).

L'hydroxygénolyse de 655 mg de désoxy-3 β(N-benzyloxycarbonyl, α-benzyl)-L-aspartylamino-3β acétoxy-12β digoxigénine en solution dans 50 ml de méthanol en présence de 160 mg de palladium à 5 % sur carbonate de calcium pendant 4 heures, permet d'obtenir après filtration, distillation à sec du filtrat et cristallisation deux fois du résidu (470 mg) dans l'éthanol absolu, 327 mg de cristaux de désoxy-3 β-L-aspartylamino-3β acétoxy-12β digoxigénine, Composé F (Rdt. 70 %).

F Kofler : 234-260 °C (dec.).

Spectre IR (Nujol) ν : 3 420, 3 250, 1 780, 1 765, 1 730, 1 640 et 1 550 $cm^{-1}$.

Spectre RMN ($CD_3OD$) δ : 0,88-1,00 et 2,08 (3s, $CH_3$) 2,87 (1H), 2,96 (2H), 4,08 (1H) 4,35 (1H), 5,10 (1H), 5,93 (1H) ppm.

CCM : ($CHCl_3$/EtOH/$NH_4OH$, 60/45/15) Rf = 0,4.

Exemple 13

Désoxy-3 γ-(N-benzyloxycarbonyl, α-benzyl)-L-glutamylamino-3β acétoxy-12β digoxigénine.

On ajoute goutte à goutte une solution de 445 mg d'acide (N-benzyloxycarbonyl, α-benzyl)-L-glutamique en solution dans 15 ml de chlorure de méthylène, à une solution, sous agitation à 0°, de 430 mg de désoxy-3 amino-3β acétoxy-12β digoxigénine et de 227 mg de dicyclohexylcarbodiimide dans 15 ml de chlorure de méthylène. Après une nuit de réaction à 4 °C, on filtre et distille le filtrat à sec. Le résidu (896 mg) est repris par le mélange acétate d'éthyle-benzène (1-1), l'insoluble est filtré et le filtrat lavé par de l'acide chlorhydrique N, de l'eau, une solution de bicarbonate de sodium, enfin de l'eau. On sèche et distille à sec la phase organique. Le résidu (793 mg) est constitué de désoxy-3 γ-N-benzyloxycarbonyl, α-benzyl)-L-aspartylamino-3β acétoxy-12β digoxigénine (Rdt. = 100 %).

Spectre IR (Nujol) : 3 480, 3 350, 1 780, 1 755, 1 730, 1 650, 1 630 et 1 525 $cm^{-1}$.

CCM ($CH_2Cl_2$/MeOH, 95/5) Rf = 0,34.

Exemple 14

Désoxy-3 γ-L-glutamylamino-3β acétoxy-12β acétoxy-12β digoxigénine (Composé G).

L'hydroxygénolyse de 680 mg de désoxy-3 γ-(N-benzyloxy carbonyl α-benzyl)-L-glutamylamino-3β acétoxy-12β digoxigénine dans 60 ml de méthanol en présence de 165 mg de palladium à 5 % sur carbonate de calcium pendant 5 heures permet d'obtenir, après filtration, distillation à sec du filtrat, lavage du résidu (503 mg) dans l'acétate d'éthyle et cristallisation par trituration dans l'éther, 320 mg de cristaux de désoxy-3 γ-L-glutamylamino-3β acétoxy-12β digoxigénine. Composé G. (Rdt. = 67 %).

F (Kofler) : 195-210° (dec.).

Spectre IR (Nujol) ν : 3 510, 3 360, 1 780, 1 765, 1 730, 1 630, 1 540 $cm^{-1}$.

Spectre RMN ($CD_3OD$) : δ : 0,92-0,99, 2,10 (3s, $CH_3$), 2,82 (1H), 1,1 (1H), 4,5 (1H), 5,04 (1H), 5,92 (1H) ppm.

**Revendications**

1. Dérivés de cardénolides représentés par la formule générale (I)

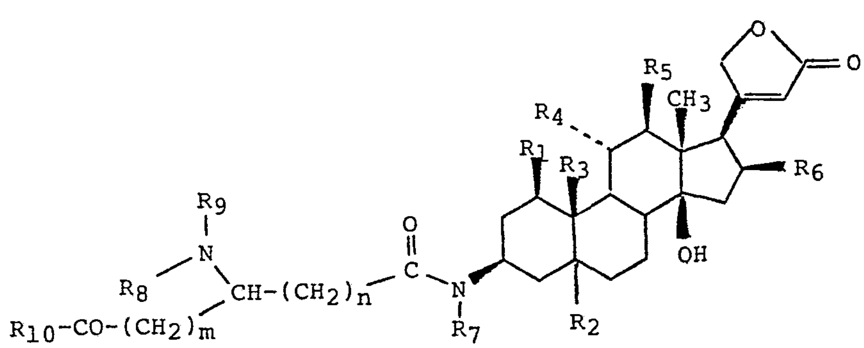

dans laquelle m et n, identiques ou différents, représentent un entier de 0 à 4, $R_1$, $R_2$, $R_4$ et $R_6$ représentent un atome d'hydrogène ou un groupe hydroxy ; $R_3$ représente un groupe méthyle, hydroxyméthyle, ou formyle ; $R_3$ représente un groupe méthyle, hydroxyméthyle, ou formyle ; $R_5$ représente un atome d'hydrogène, un groupe hydroxy ou un groupe acétoxy ; $R_7$ représente un atome d'hydrogène ou un groupe alkyle inférieur de 1 à 5 atomes de carbone ; $R_8$ représente un atome d'hydrogène, un groupe alkyle inférieur de 1 à 5 atomes de carbone, acyle, alkyloxycarbonyle, ou

aralcoxycarbonyle ; $R_9$ représente un atome d'hydrogène ou un groupe alkyle inférieur de 1 à 5 atomes de carbone ; $R_8$ et $R_9$ peuvent former un hétérocycle conjointement avec l'atome d'azote ; $R_{10}$ représente un groupe hydroxy, alcoxy, ou aralcoxy ; ainsi que leurs sels de bases ou d'acides minéraux et organiques.

2. Dérivés de cardénolides selon la revendication 1, caractérisés en ce que $R_7$ et $R_9$ représentent un atome d'hydrogène ou un groupe méthyle ; et $R_8$ représente un atome d'hydrogène, un groupe méthyle, un groupe alkyloxycarbonyle, ou aralcoxy-carbonyle.

3. Dérivés de cardénolides selon l'une quelconque des revendications 1 et 2, caractérisés en ce que n est égal à 1 ou 2, et m est égal à 0.

4. Dérivés de cardénolides selon l'une quelconque des revendications 1 et 2, caractérisés en ce que n est égal à 0, et m est égal à 1 ou 2.

5. Dérivés de cardénolides selon la revendication 1, caractérisés en ce qu'ils sont choisis parmi le groupe constitué par la désoxy-3 α-L-aspartylamino-3β digitoxigénine, la désoxy-3 β-L-aspartylamino-3β digitoxigénine, la désoxy-3 γ-L-glutamylamino-3β digitoxigénine, la désoxy-3 N-β-L-aspartyl n'-méthylamino-3β digitoxigénine, et la désoxy-3 N-γ-L-glutamyl N'-méthylamino-3β digitoxigénine.

6. Dérivés de cardénolides selon la revendication 1, caractérisés en ce qu'ils sont choisis parmi la désoxy-3 β-L-aspartylamino-3β acétoxy-12β digoxigénine et la désoxy-3 γ-L-glutamylamino-3β acétoxy-12β digoxigénine.

7. Compositions pharmaceutiques caractérisées en ce qu'elles contiennent, à titre de principe actif, un dérivé de cardénolide selon l'une quelconque des revendications 1 à 6, associé à un ou plusieurs excipients pharmaceutiquement acceptables, et, le cas échéant, à un ou plusieurs autres principes actifs.

8. Procédé de préparation des aminocardénolides selon la revendication 1, caractérisé en ce que l'on fait réagir un aminocardénolide préalablement protégé et un dérivé de diacide aminé convenablement substitué au niveau du groupe amino et au niveau de l'une de deux fonctions acides carboxylique, l'autre étant activée, et le cas échéant on élimine les groupes protecteurs, puis on effectue éventuellement une salification.

9. Procédé de préparation selon la revendication 8, caractérisé en ce que la réaction est effectuée à température ambiante.

## Claims

1. Cardenolide derivatives represented by the general formula (I)

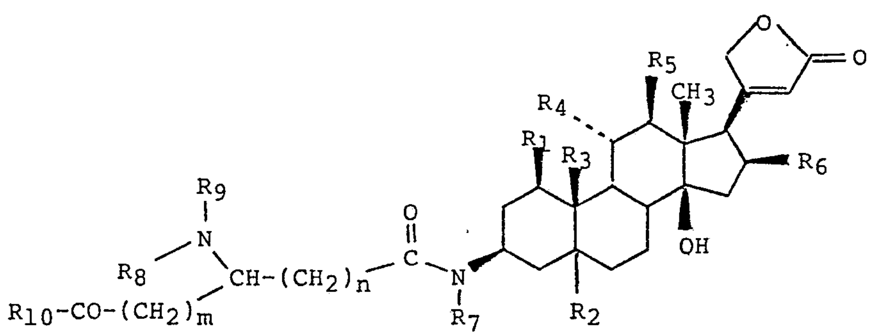

wherein m and n, which may be the same or different, represent an integer from 0 to 4 ; $R_1$, $R_2$, $R_4$ and $R_6$ represent a hydrogen atom or a hydroxy group ; $R_3$ represents a methyl, hydroxymethyl or formyl group ; $R_5$ represents a hydrogen atom, a hydroxy or acetoxy group ; $R_7$ represents a hydrogen atom or a lower alkyl group from 1 to 5 carbon atoms ; $R_8$ represents a hydrogen atom or a lower alkyl group from 1 to 5 carbon atoms, acyl, alkyloxycarbonyl or aralkoxycarbonyl group ; $R_9$ represents a hydrogen atom or a lower alkyl group from 1 to 5 carbon atoms ; $R_8$ and $R_9$ can combine with the nitrogen atom and form a heterocyclic ring ; $R_{10}$ represents a hydroxy, alkoxy, or aralkoxy group, and mineral and organic basic or acid salts thereof.

2. The cardenolide derivatives according to claim 1, characterized in that $R_7$ and $R_9$ represent a hydrogen atom or a methyl group ; and $R_8$ represents a hydrogen atom, a methyl group or an alkoxycarbonyl or aralkoxycarbonyl group.

3. The cardenolide derivatives according to any of claims 1 and 2, characterized in that n is 1 or 2 and m is 0.

4. The cardenolide derivatives according to any of claims 1 and 2, characterized in that n is 0 and m is 1 or 2.

5. The cardenolide derivatives according to claim 1, characterized in that they are selected from the group consisting of deoxy-3 α-L-aspartylamino-3β digitoxigenin, deoxy-3 β-L-aspartylamino-3β digitoxigenin, deoxy-3 γ-L-glutamylamino-3β digitoxigenin, deoxy-3 N-β-L-aspartyl-N'-methylamino-3β digitoxigenin, and deoxy-3 N-γ-L-glutamyl N'-methylamino-3β digitoxigenin.

6. The cardenolide derivatives according to claim 1, characterized in that they are selected from the group consisting of deoxy-3 β-L-aspartylamino-3β acetoxy-12β digoxigenin, and deoxy-3 N-γ-L-glutamylamino-3β acetoxy-12β digoxigenin.

7. Pharmaceutical compositions characterized in that they comprise, as an active ingredient, a cardenolide derivative according to any of claims 1 to 6 with one or more pharmaceutically acceptable carriers or diluents, and, possibly, one or more other active principles.

8. A process for preparation of aminocardenolides according to claim 1, characterized in that it comprises reacting an aminocardenolide previously protected with an amino diacid derivative suitably substituted at the amino functional group, and at one of the two carboyxlic acid functional groups thereof, the other of said carboxylic acid functional groups being activated, and if necessary, removing the protective groups and, if necessary, forming a salt thereof.

9. The process according to claim 8, wherein the reaction is carried out at room temperature.

**Ansprüche**

1. Cardenolidderivate der allgemeinen Formel (I)

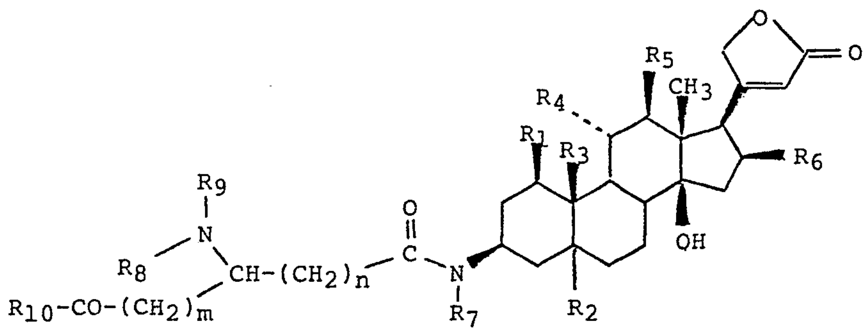

in der n und m, die gleichartig oder verschieden sein können, die Werte 0, 1, 2, 3 oder 4 haben können und $R_1$, $R_2$, $R_4$ und $R_6$ ein Wasserstoffatom oder eine Hydroxygruppe, $R_3$ eine Methyl gruppe, eine Hydroxymethylgruppe oder eine Formylgruppe, $R_5$ ein Wasserstoffatom, eine Hydroxygruppe oder eine Acetoxygruppe, $R_7$ ein Wasserstoffatom oder eine niedere Alkylgruppe mit 1 bis 5 Kohlenstoffatomen, $R_8$ ein Wasserstoffatom, eine niedere Alkylgruppe mit 1 bis 5 Kohlenstoffatomen, eine Acylgruppe, eine Alkoxycarbonylgruppe oder eine Aralkoxycarbonylgruppe, $R_9$ ein Wasserstoffatom oder eine niedere Alkylgruppe, mit 1 bis 5 Kohlenstoffatomen, $R_8$ and $R_9$ gemeinsam mit dem Stockstoffatom eine heterocyclische Gruppe, $R_{10}$ eine Hydroxygruppe, eine Alkoxygruppe oder eine Aralkoxygruppe bedeuten, sowie die anorganischen oder organischen Salze dieser Derivate.

2. Cardenolidderivate nach Anspruch 1, dadurch gekennzeichnet, dass $R_7$ und $R_9$ ein Wasserstoffatom oder eine Methylgruppe, und $R_8$ ein Wasserstoffatom oder eine Methylgruppe, eine Alkoxycarbonylgruppe oder eine Aralkoxycarbonylgruppe bedeuten.

3. Cardenolidderivate nach einem der Ansprüche 1 oder 2, dadurch gekennzeichnet, dass n die Werte 1 oder 2, und m den Wert 0 besitzen.

4. Cardenolidderivate nach einem der Ansprüche 1 oder 2, dadurch gekennzeichnet, dass m die Werte 1 oder 2 and n den Wert 0 besitzen.

5. Cardenolidderivate nach Anspruch 1, dadurch gekennzeichnet, dass sie aus Desoxy-3 α-L-Aspartyl-amino-3β Digitoxigenin, Desoxy-3 β-L-Aspartylamino-3β Digitoxigenin, Desoxy-3 γ-L-Glutamyl-amino-3β Digitoxigenin, Desoxy-3 N-β-L-Aspartyl N'-Methylamino-3β Digitoxigenin, oder Desoxy-3 N-γ-L-Glutamyl N'-Methyl-amino-3β Digitoxigenin bestehen.

6. Cardenolidderivate nach Anspruch 1, dadurch gekennzeichnet, dass sie aus Desoxy-3 β-L-Aspartylamino-3β Acetoxy-12β Digoxigenin oder Desoxy-3 γ-L-Glutamylamino-3β Acetoxy-12β Digoxigenin bestehen.

7. Pharmazeutische Zubereitungen, dadurch gekennzeichnet, dass sie als Wirkstoff ein Cardenolidderivat nach einem der Ansprüche 1 bis 6, sowie ein oder mehrere pharmazeutisch verträglichen Trägermateriale und/oder andere Wirkstoffe enthalten.

8. Verfahren zur Herstellung der Cardenolidderivate nach Anspruch 1, dadurch gekennzeichnet, dass man eine Reaktion zwischen dem zuvor geschützten Aminocardenolid und einem Dicarbonsäurederivat, deren Carbonsäuregruppe und deren Aminogruppe in geeigneter Weise substituiert sind, und deren anderen Carbonsäuregruppe aktiviert ist, durchführt und gegebenenfalls die Schutzgruppen abgespalten sind, und gegebenenfalls das erhaltene Produkt mit einer anorganischen oder organischen Base oder Saüre in ein Salz überführt.

9. Verfahren nach Anspruch 8, dadurch gekennzeichnet, dass man die Reaktion bei Raumtemperatur ablaufen lässt.